(19) Europäisches Patentamt
European Patent Office
Office européen des brevets

(11) Numéro de publication: **0 189 696 A1**

(12) # DEMANDE DE BREVET EUROPEEN

(21) Numéro de dépôt: 85402522.8

(22) Date de dépôt: 17.12.85

(51) Int. Cl.⁴: A 61 K 31/725
//(A61K31/725, 31:19)

(30) Priorité: 17.12.84 FR 8420129

(43) Date de publication de la demande:
06.08.86 Bulletin 86/32

(84) Etats contractants désignés:
AT BE CH DE FR GB IT LI LU NL SE

(71) Demandeur: SANOFI
40, Avenue George V
F-75008 Paris(FR)

(71) Demandeur: SCLAVO S.p.A.
Via Fiorentina 1
I-53100 Siena(IT)

(72) Inventeur: Bayol, Alain
5 rue de la Sardane
F-31170 Tournefeuille(FR)

(72) Inventeur: Blanc, Francis
Chemin de Soriech
F-34000 Lattes(FR)

(72) Inventeur: Lansen, Jacqueline
120, Rue des Chardonnerets St. Clément la Rivière
34980 St. Gely du Fesc(FR)

(72) Inventeur: Maffrand, Jean-Pierre
5 rue du Corps-Franc Pommiès
F-31120 Portet/Garonne(FR)

(72) Inventeur: Pereillo, Jean-Marie
9 avenue des Mimosas
F-31120 Portet/Garonne(FR)

(74) Mandataire: Polus, Camille et al,
c/o Cabinet Lavoix 2, Place d'Estienne d'Orves
F-75441 Paris Cedex 09(FR)

(54) **Nouvelle composition thérapeutique à activité antithrombotique et hypolipémiante.**

(57) La présente invention a pour objet une composition thérapeutique contenant, à titre de principe actif, des sulfates de xylanes et un acide organique.

Cette composition presente une puissante activité antithrombotique et hypolipémiante due à un effet de synergie entre ses deux constituants.

EP 0 189 696 A1

La présente invention concerne une composition thérapeutique possédant des activités antithrombotique et hypolipémiante, contenant, à titre de principe actif, une association de sulfates de xylanes et d'un acide organique.

Les sulfates de xylanes sont constitués, soit par le polysulfate de xylane, appelé SP 54 ou PZ 68 selon les auteurs, soit par une des différentes fractions isolées à partir du SP 54. Le SP 54 est un polysulfate de xylane obtenu par sulfatation ménagée de xylanes extraits de bois de hêtre. Ce produit est commercialisé depuis une vingtaine d'années pour utilisation par voie injectable, notamment dans la prévention des thromboses veineuses post-opératoires, et par voie orale, notamment dans le traitement des dyslipémies athérogènes.

Par ses effets sur les systèmes de coagulation (FISCHER A. M., BARROWCLIFFE T. W. THOMAS D. P. Thromb Haemost, 1982, 47, (2), 104) (FISCHER A. M., MERTON R. E., MARSH N. A., WILLIAMS S., GAFFNEY P. J., BARROWCLIFFE T. W. & THOMAS D. P. Thromb Haemost, 1982, 47, (2), 109) (SCULLY M. F., WEERASINGHE K. M., ELLIS V., DJAZAERI B. & KAKKAR V. V. Thromb Res, 1983, 31, 87) et de fibrinolyse (VINAZZER H., STEMBERGER A., HAAS S. & BLÜMEL G., Thromb Res, 1982, 27, 341), sur le système complémentaire (WALB D., LOOS M. & HADDING U. 2 : Natusforsch, 1971, 26, 403), sur la paroi des vaisseaux (PAUL R., HERBERT J. M., MAFFRAND J. P., LANSEN J. & RONCUCCI R., Symposium on Atheroma and Thrombosis, London, July 1983, et sur le métabolisme lipidique (ROUFFY J., Mises à jour Cardiol, 1983, 12 (8), 381, ce produit présente, en effet, des propriétés antithrombotique et antiathéromateuse intéressantes.

Il se présente sous la forme d'un mélange de polymères (sulfates de xylanes) de différents poids moléculaires. La définition analytique de tels mélanges est toujours délicate et plusieurs expérimentateurs ont

pu donner des résultats différents pour un même produit.

Ainsi, VINAZZER et al. ont reporté pour le SP 54 un poids moléculaire de 2000 (Thromb. Res., 1980, 20, 57-68), puis de 3000 (Thromb. Res., 1982, 27, 341-352) ; C. D. ESQUIVEL et al. (Thromb. Res., 1982, 28, 389-399) ont décrit un poids moléculaire moyen de 4000 ; enfin, C. SORIA et al. (Thromb. Res., 1980, 19, 455-463) ont signalé un poids moléculaire moyen de 6000 qui a été aussi publié très récemment par M. F. SCULLY et V. V. KAKKAR (Biochem. J., 1984, 218, 657).

Cette disparité dans les poids moléculaire moyens est, sans doute, due aux méthodes d'analyse et aux standards utilisés, ainsi qu'à l'absence de précision sur la nature (apparente, en poids, en nombre) de la masse molaire publiée.

A l'aide des techniques décrites plus loin, la Demanderesse a trouvé que le SP 54 ou PZ 68, qui répond à la formule statistique développée suivante :

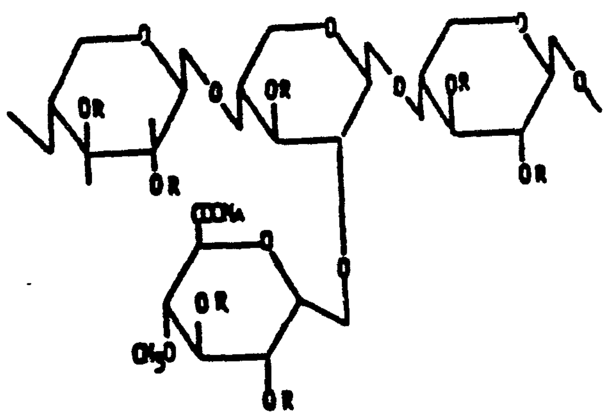

I

dans laquelle R représente le groupe $-SO_3$ Na ou l'hydrogène, peut être défini de la manière suivante :

Il s'agit d'un mélange de polymères de masses molaires comprises entre 1000 et 40000 Daltons, présentant une distribution quasi-gaussienne.

Les mesures de paramètres de distribution moléculaires de ce produit, par chromatographie d'exclusion haute performance, font apparaître pour différents

lots de fabrication des valeurs moyennes de :

Masse moléculaire moyenne en nombre ou $\overline{Mn}$ : 5300 à 5500,

Masse moléculaire moyenne en poids ou $\overline{Mw}$ : 7400 à 7600,

Indice de polydispersité ou $D = \frac{Mw}{Mn}$ mesurant la largeur de la distribution moléculaire : 1,4 à 1,5

La masse moyenne apparente ou $\overline{Ma}$ qui correspond à la masse moléculaire de la population isomoléculaire majoritaire en concentration (c'est-à-dire le sommet du pic sur le chromatogramme) est comprise entre 6500 et 7200 Daltons. Le taux de sulfatation du produit, c'est-à-dire le nombre de fonctions hydroxyle estérifiées par un groupement sulfurique est accessible par le dosage pondéral moyen du soufre : 15 à 20 %.

On peut définir aussi le degré de sulfatation moyen de ce mélange de polymères ou D. S., comme le nombre moyen de groupes sulfuriques par unité xylose considéré comme élément monomère.

Le degré de sulfatation moyen pour le produit, accessible par résonance magnétique nucléaire du proton ou par dosages centésimaux microanalytiques du carbone et du soufre est compris entre 1,5 et 2, et plus précisément proche de 1,8.

Ce polysulfate de xylane est aussi caractérisé par les valeurs moyennes des dosages de pentoses et d'acides glucuroniques.

Ainsi, le pourcentage pondéral de pentoses, exprimé en équivalent xylose est de 30 à 40 %.

Le pourcentage pondéral d'acides uroniques (en fait principalement acide 4 MeO-glucuronique), exprimé en acide glucuronique varie de 4 à 6 %.

Ceci correspond à une proportion de 1 unité glucuronique pour 8 à 10 unités xyloses.

Différentes fractions isolées à partir du

SP 54, ainsi que leur préparation, ont été décrites par la Demanderesse dans les demandes de brevet N° 83.05.170 du 24.03.1983 et N° 84.17.460 du 07.11.1984.

Ces fractions peuvent être définies par les caractéristiques suivantes :

- degré de sulfatation moyen compris entre 1,5 et 2,
- masses moléculaires moyennes apparentes $\overline{Ma}$ comprises entre 2000 et 12000 Daltons,
- taux pondéral de pentoses exprimés en xylose compris entre 30 et 40 %,
- taux pondéral d'acides uroniques exprimé en acide glucuronique compris entre 0 et 10 %,
- les indices de polydispersité des distributions moléculaires pour les produits de l'invention sont inférieures ou égales à celles du produit.

Ces caractéristiques ont été déterminées selon les méthodes ci-après :

1 - <u>Spectre de résonance magnétique nucléaire du carbone 13</u>

Le spectre de résonance magnétique nucléaire du carbone 13 (80 MHz) est réalisé sur une solution à 10 % (m/V) dans l'eau deutériée. Les déplacements chimiques des pics correspondant aux atomes de carbone de l'unité xylose sulfatée, sont les suivants : $C_1$ : 100,4 ppm ; $C_2$ : 73,6 ppm ; $C_3$ : 74,1 ppm ; $C_4$ : 75,43 ppm et $C_5$ : 60,3 ppm.

2 - <u>Spectre de résonance magnétique nucléaire du proton</u>

Le spectre de résonance magnétique nucléaire du proton (250 $MH_2$) est réalisé à la concentration de 20 % (m/V) dans l'eau deutériée. Le signal correspondant à l'eau est déplacé par ajout d'acide trifluoroacétique. Le degré de sulfatation peut être calculé sur la base de l'intégration des protons 1 et 3 (5,2 ppm et 4,8 ppm) de l'unité xylose sulfatée.

3 - Dosage des groupements méthoxy

La méthode employée est celle décrite par LAVER M. et WOLFROM M. L. dans Methods in Carbohydrate Chemistry, Vol. 1, page 454, Edité par Ac. Press. 1962.

4 - Dosage des acides uroniques

La méthode employée est celle au carbazole de BITTER T. et MUIR H. M. (Anal Biochem, 1962, 4, 330-334).

5 - Microdosage du soufre

Après minéralisation en fiole de SHONIGER W. (Mikrochim Acta. 1956, 1, 869-876), le soufre est dosé conductrimétriquement par le perchlorate de baryum.

Les pourcentages centésimaux de soufre et de carbone (obtenu par microanalyse) permettent de calculer de degré de sulfatation ou D. S. :

- nombre de moles de soufre pour 100 g du produit :

$$\frac{\%\ S}{32{,}06}$$

- nombre de moles de carbone pour 100 g du Produit :

$$\frac{\%\ C}{12{,}011}$$

- nombre de moles de xylose pour 100 g du Produit :

$$\frac{\%\ C}{12{,}011 \times 5}$$

- nombre moyen de fonctions sulfates par xylose monomère ou

$$D.S. = 5 \times \frac{\%\ S}{\%\ C} \times \frac{12{,}011}{32{,}06}$$

6 - Dosage des pentoses

La méthode employée est celle à l'orcinol et au chlorure ferrique en milieu chorhydrique (MEJBAUM W. - Z. Physiol. Chem., 1979, 258, 117) en prenant le xylose comme étalon.

7 - Dosage du sulfate de sodium

Il est réalisé par chromatographie liquide haute performance en utilisant une colonne IONOSPHER TmA de 25 cm de longueur (CHROMPACK réf. 28300) avec comme éluant de l'hydrogène phtalate de potassium à 0,7 % (m/V) dans l'eau distillée. L'étalonnage est réalisé avec du sulfate de sodium anhydre.

8 - Détermination de la masse moléculaire

Elle est réalisée par chromatographie d'exclusion à travers deux colonnes de silice de porosité 60 Å et 500 Å placées en série. L'appareil utilisé est celui de HEWLETT PACKARD 1081 B équipé d'un réfractomètre JOBIN et YVON IOTA et d'un injecteur HP 79841 A. L'ensemble est thermostaté à 30° C. La phase mobile contenant du sulfate de sodium est préparée selon BARTH H. G. et SMITH D. A. (J. Chromatogr. 1981, 206, 410-415) et modifiée en ajoutant 0,5 % de polyéthylène glycol pour éviter les interactions de type pont hydrogène. La foce ionique de l'éluant est égale à 1.

Les masses moléculaires moyennes en nombre ($\overline{Mn}$), en poids ($\overline{Mw}$) et apparente (Ma) sont calculées à partir d'un étalonnage réalisé à l'aide d'oligosaccharides neutres (xylose, maltotriose) et de Dextranes (T40-T70 et T500) selon la méthode décrite par YAU W. W. ; KIRLAND J. J. et BLY D. D. dans Modern size-exclusion Liquid Chromatography page 315 Edité par J. WILEY et DONS (1979).

L'indice de polydispersité est calculé selon le rapport $\overline{Mw}/\overline{Mn}$.

Parmi les fractions utilisables, à titre de constituant du principe actif, dans la composition thérapeutique de l'invention, on citera :

| FRACTION | CARACTERISTIQUES ANALYTIQUES | | | |
|---|---|---|---|---|
| | MN | MW | D | Ma |
| 1 | 2794 | 3380 | 1,21 | 3346 |
| 2 | 2732 | 4183 | 1,53 | 3518 |
| 3 | 3330 | 4480 | 1,34 | 3889 |
| 4 | 3307 | 4013 | 1,33 | 3939 |
| 5 | 3133 | 4716 | 1,50 | 4066 |
| 6 | 3470 | 4570 | 1,30 | 4407 |
| 7 | 3648 | 4958 | 1,36 | 4674 |
| 8 | 4039 | 5058 | 1,25 | 4903 |
| 9 | 3680 | 5585 | 1,52 | 4921 |
| 10 | 6700 | 7850 | 1,17 | 7500 |
| 11 | 7180 | 9500 | 1,32 | 7890 |

Le SP 54 ainsi que les fractions telles que définies ci-dessus, sont doués d'importantes activités anti-thrombotique et hypolipémiante.

La Demanderesse a maintenant trouvé que, lorsqu'on administre le SP 54 ou une de ses fractions, en association avec un acide organique pharmaceutiquement acceptable, de préférence un acide di- ou polycarboxylique et notamment l'acide citrique, isocitrique, tartrique, malique ou ascorbique, on obtient une augmentation considérable des activités anti-thrombotique et hypolipémiante. Ainsi, l'association constituant le principe actif de la composition thérapeutique suivant l'invention, présente un effet de synergie qui va être mis en évidence dans l'étude pharmacologique suivante.

Cette étude a porté sur les actions anticoagulante, fibrinolytique et lipolytique par les voies orale et intraduodénale, produites par l'association de l'invention par comparaison avec le SP 54 et ses différentes fractions, utilisés seuls.

- L'action anticoagulante est déterminée par le temps de céphaline activée (CAEN J., LARRIEU M. J., SAMAMA M., In : L'hémostase : méthode d'exploration et diagnostic pratique. Ed. : l'Expansion Scientifique Française, 1975, p. 169).
Le temps de céphaline activée (TCA) mesure la recalcification du plasma déplaquetté en présence d'un optimum de lipides (céphaline) et de célite qui active de manière standardisée les facteurs de la phase de contact (les facteurs XII, XI, IX). Il mesure donc la formation de prothrombinase endogène à l'exception des facteurs plaquettaires remplacés par la céphaline.

- L'activité fibrinolytique est mesurée par le temps de lyse des euglobulines plasmatiques (KLUFT C. - Haemostasis, 1976, 5, 136).
Le temps de lyse des euglobulines plasmatiques est une méthode sensibilisée permettant d'apprécier l'action fibrinolytique globale du SP 54 et de ses fractions en l'absence des inhibiteurs physiologiques de la fibrinolyse, éliminés par dilution et acidification du plasma.

- L'action lipolytique est mesurée par l'activité lipoprotéine lipase circulante (NIKKILA E. A., HUTTUNEN J. K., EHNHOLM C., - Metabolism, 1977, 26, 179).
La détermination de l'activité lipoprotéine lipase

circulante est une mesure indirecte de l'impact du SP 54 et de ses fractions sur le catabolisme des triglycérides (pouvoir clarifiant). L'activité enzymatique est déterminée par sa capacité d'hydrolyser le substrat 14C-trioléine en 14C-acide oléique.
Le SP 54 et ses différentes fractions sont administrés seuls, en une dose unique de 400 mg, puis en association avec l'acide citrique aux doses de 30 mg/kg pour la voie intraduodénale et 120 mg/kg pour la voie orale.

- <u>Administration intraduodénale et orale</u> des rats mâles (Sprague Dawley ; Charles River ; France) de 200 à 300 g sont utilisés. Les animaux sont soumis à une diète hydrique la veille des essais.
Une anesthésie au pentobarbital (50 mg/kg ; i. p.) est effectuée sur tous les animaux avant l'administration des différents produits à tester.
Pour l'administration intraduodénale, une incision est effectuée au niveau de l'abdomen. Le pylore et le duodénum sont isolés. Une incision est pratiquée à 1 cm du pylore et les produits sont introduits à l'aide d'une sonde dans le duodénum. Une ligature est effectuée en dessous de l'incision à l'aide de fil chirurgical. L'administration orale est effectuée par gavage à l'aide d'une sonde intragastrique.
Les prélèvements sanguins pour l'analyse des divers paramètres pharmacologiques sont réalisés à l'aorte abdominale respectivement 1 heure après l'administration intraduodénale et 2 heures après l'administration orale. Ils sont rendus incoagulables par une solution de citrate trisodique à 3,8 p. 100 (1 volume d'anticoagulant pour 9 volumes de sang) ou de l'héparine (250 U/ml de sang) pour les mesures de lipoprotéine lipase.
Immédiatement après le prélèvement sanguin, les échantillons sont centrifugés à 2000 x g pendant 15 minutes à 4°C, pour la préparation du plasma citraté qui est

utilisé pour les différents dosages.
Les résultats sont rassemblés dans les tableaux 1 à 5 ; leur analyse statistique a été effectuée selon le test de STUDENT (groupe de comparaison : SP 54).

TABLEAU 1

VOIE INTRADUODENALE - TEMPS de CEPHALINE ACTIVEE

| Produit | Ma | 400 mg/kg | | | | 400 mg/kg + 30 mg/kg d'acide citrique | | | |
|---|---|---|---|---|---|---|---|---|---|
| | | M± ESM sec | % (a) | test "t" vs | | M± ESM sec | % (a) | test "t" vs | |
| | | | | Tem | SP54 | | | Tem | SP54 |
| TEMOINS | | 20 ± 0,6 | -- | -- | n.s. | 20 ± 0,4 | -- | -- | p<0,001 |
| Fraction 1 | 3346 | 32 ± 1,5 | 60 | p<0,001 | p<0,001 | 68 ± 5 | 240 | p<0,001 | p<0,001 |
| Fraction 2 | 3518 | 32 ± 1,6 | 60 | p<0,001 | p<0,001 | 59 ± 0,5 | 195 | p<0,001 | p<0,001 |
| Fraction 3 | 3889 | 24 ± 0,5 | 20 | p<0,01 | p<0,01 | 47 ± 4 | 135 | p<0,01 | p<0,01 |
| Fraction 5 | 4066 | 28 ± 0,9 | 40 | p<0,001 | p<0,001 | 52 ± 1,7 | 160 | p<0,001 | p<0,001 |
| Fraction 6 | 4407 | 29 ± 1,9 | 45 | p<0,01 | p<0,01 | 47 ± 2 | 135 | p<0,001 | p<0,001 |
| Fraction 8 | 4903 | 24 ± 0,7 | 20 | p<0,01 | p<0,05 | 43 ± 1,5 | 115 | p<0,001 | p<0,01 |
| Fraction 9 | 4921 | 29 ± 0,7 | 45 | p<0,001 | p<0,001 | 45 ± 1,2 | 125 | p<0,001 | p<0,001 |
| Fraction 10 | 7500 | 22 ±0,9 | 10 | p<0,05 | n.s. | 51 ± 4 | 155 | p<0,001 | p<0,01 |
| Fraction 11 | 7890 | 21 ±0,8 | 5 | n.s. | n.s. | 58 ± 0,4 | 190 | p<0,001 | p<0,01 |
| SP 54 | 6600 | 21 ± 0,4 | 5 | n.s. | -- | 32 ± 1,2 | 60 | p<0,001 | -- |

(a) : % prolongation

## TABLEAU 2

**VOIE INTRADUODENALE - ACTIVITE ANTI-Xa (TEMPS DE VIN)**

| Produit | Ma | 400 mg/kg | | | | 400 mg/kg + 30 mg/kg d'acide citrique | | | |
|---|---|---|---|---|---|---|---|---|---|
| | | M ± ESM sec | % (a) | test "t" vs Tem | test "t" vs SP54 | M ± ESM sec | % (a) | test "t" vs Tem | test "t" vs SP54 |
| TEMOINS | | 33 ± 0,6 | -- | -- | n.s. | 33 ± 0,6 | -- | -- | p<0,001 |
| Fraction 1 | 3346 | 36 ± 0,5 | 9 | p<0,05 | p<0,05 | 43 ± 1,4 | 30 | p<0,001 | p<0,001 |
| Fraction 2 | 3518 | 36 ± 0,3 | 9 | p<0,05 | p 0,05 | 46 ± 0,7 | 39 | p 0,001 | p 0,01 |
| Fraction 3 | 3889 | 33 ± 0,3 | 0 | n.s. | n.s. | 40 ± 1,5 | 21 | p<0,01 | p<0,05 |
| Fraction 5 | 4066 | 38 ± 0,0 | 15 | p<0,01 | p 0,05 | 46 ± 1,4 | 39 | p<0,001 | p<0,04 |
| Fraction 6 | 4407 | 35 ± 1,9 | 6 | n.s. | n.s. | 41 ± 0,7 | 24 | p<0,001 | p<0,01 |
| Fraction 8 | 4903 | 33 ± 0,7 | 0 | n.s. | n.s. | 39 ± 1,1 | 18 | p<0,01 | p<0,05 |
| Fraction 9 | 4921 | 37 ± 0,4 | 12 | p<0,05 | p 0,05 | 42 ± 0,6 | 27 | p<0,001 | p<0,01 |
| Fraction 10 | 7500 | 35 ± 0,6 | 0 | n.s. | n.s. | 43 ± 1 | 23 | p<0,001 | p<0,01 |
| Fraction 11 | 7890 | 34 ± 0,2 | 0 | n.s. | n.s. | 38 ± 0,5 | 19 | p<0,001 | p<0,01 |
| SP 54 | 6600 | 34 ± 1 | 3 | n.s. | -- | 37 ± 0,6 | 12 | p<0,001 | -- |

(a) : % prolongation

0189696

TABLEAU 3

VOIE INTRADUODENALE - TEMPS DE LYSE DES EUGLOBULINES

| Produit | Ma | 400 mg/kg | | | | 400 mg/kg + 30 mg/kg d'acide citrique | | | |
|---|---|---|---|---|---|---|---|---|---|
| | | M±ESM sec | % (a) | test "t" vs | | M± ESM sec | % (a) | test "t" vs | |
| | | | | Tem | SP54 | | | Tem | SP54 |
| TEMOINS | | 134 ± 6 | -- | -- | n.s. | 134 ± 2 | -- | -- | p<0,001 |
| Fraction 1 | 3346 | 102 ± 4 | 24 | p<0,01 | p<0,05 | 74 ± 7 | 45 | p<0,001 | n.s. |
| Fraction 2 | 3518 | 97 ± 4 | 28 | p<0,001 | p<0,001 | 74 ± 7 | 45 | p<0,001 | n.s. |
| Fraction 3 | 3889 | 105 ± 4 | 22 | p<0,01 | p<0,05 | 94 ± 4 | 30 | p<0,001 | n.s. |
| Fraction 5 | 4066 | 93 ± 4 | 30 | p<0,01 | p<0,01 | 71 ± 6 | 47 | p<0,001 | n.s. |
| Fraction 6 | 4407 | 72 ± 2 | 46 | p<0,001 | p<0,001 | 95 ± 3 | 29 | p<0,001 | n.s. |
| Fraction 8 | 4903 | 94 ± 8 | 30 | p<0,01 | p<0,05 | 111 ± 7 | 17 | p<0,05 | p<0,05 |
| Fraction 9 | 4921 | 99 ± 5 | 26 | p<0,01 | p<0,01 | 71 ± 7 | 47 | p<0,001 | n.s. |
| Fraction 10 | 7500 | 106±2 | 21 | p<0,01 | p<0,05 | 85 ± 4 | 37 | p<0,001 | n.s. |
| Fraction 11 | 7890 | 95 ± 9 | 29 | p<0,01 | p<0,05 | 84 ± 3 | 37 | p<0,001 | n.s. |
| SP 54 | 6600 | 124 ± 8 | 7 | n.s. | -- | 84 ± 2 | 37 | p<0,001 | -- |

. (a) : % d'activation

TABLEAU 4

VOIE INTRADUODENALE - LIPOPROTEINE LIPASE (L.P.L.)

| Produit | Ma | 400 mg/kg | | | | 400 mg/kg + 30 mg/kg d'acide citrique | | | |
|---|---|---|---|---|---|---|---|---|---|
| | | M ± ESM × | % (a) | test "t" vs Tem | test "t" vs SP54 | M ± ESM sec | % (a) | test "t" vs Tem | test "t" vs SP54 |
| TEMOINS | | 3,2 ± 0,3 | 6 | -- | p<0,001 | 5 ± 0,2 | 8 | -- | p<0,001 |
| Fraction 1 | 3346 | 14,1 ± 1,6 | 26 | p<0,001 | p<0,01 | 40 ± 1,7 | 64 | p<0,001 | n.s. |
| Fraction 2 | 3518 | 15 ± 2 | 28 | p<0,001 | p<0,01 | 55,2 ± 4,4 | 88 | p<0,001 | p<0,001 |
| Fraction 3 | 3889 | 7,1 ± 0,8 | 13 | p<0,001 | n.s. | 27,5 ± 1,9 | 44 | p<0,001 | p<0,001 |
| Fraction 5 | 4066 | 15 ± 0,65 | 28 | p<0,001 | p<0,01 | 33,9 ± 6,9 | 54 | p<0,001 | n.s. |
| Fraction 6 | 4407 | 3,9 ± 0,7 | 7 | n.s. | p<0,01 | 20,7 ± 5,2 | 33 | p<0,001 | p<0,05 |
| Fraction 8 | 4903 | 6,7 ± 1,1 | 16 | p<0,001 | n.s. | 30,4 ± 4,9 | 49 | p<0,001 | p<0,05 |
| Fraction 9 | 4921 | 15,1 ± 1,5 | 28 | p<0,001 | p<0,01 | 39,4 ± 1,9 | 63 | p<0,001 | n.s. |
| Fraction 10 | 7500 | 6,6 ± 1,0 | 11 | p<0,001 | n.s. | 43,2 ± 0,6 | 71 | p<0,001 | n.s. |
| Fraction 11 | 7800 | 9,9 ± 1,0 | 16 | p<0,001 | n.s. | 34,2 ± 3,1 | 56 | p<0,001 | p<0,05 |
| SP 54 | 6600 | 7,6 ± 0,6 | 14 | p<0,001 | -- | 47,3 ± 1,8 | 76 | p<0,001 | -- |

(a) : % de libération

× mol acide oléique/ml/h

TABLEAU 5

VOIE ORALE - TEMPS DE CEPHALINE ACTIVEE

| Produit | Ma | 400 mg/kg | | | | 400 mg/kg + 120 mg/kg d'acide citrique | | | |
|---|---|---|---|---|---|---|---|---|---|
| | | M ± ESM sec | % (a) | test "t" vs Tem | test "t" vs SP54 | M ± ESM sec | % (a) | test "t" vs Tem | test "t" vs SP54 |
| TEMOINS | | 20 ± 0,4 | -- | -- | -- | 19 ± 0,4 | -- | -- | -- |
| Fraction 1 | 3346 | 29 ± 0,5 | 45 | p<0,001 | p<0,001 | 34 ± 0,7 | 79 | p<0,001 | p<0,001 |
| Fraction 2 | 3518 | 31 ± 0,6 | 55 | p<0,001 | p<0,001 | 40 ± 1,8 | 111 | p<0,001 | p<0,001 |
| Fraction 3 | 3889 | 21 ± 0,8 | 5 | n.s. | n.s. | 23 ± 0,1 | 21 | p<0,01 | p<0,001 |
| Fraction 5 | 4066 | 29 ± 0,6 | 45 | p<0,001 | p<0,001 | 29 ± 0,9 | 53 | p<0,001 | n.s. |
| Fraction 6 | 4407 | 21 ± 0,3 | 5 | n.s. | n.s. | 22 ± 0,4 | 16 | p<0,01 | p<0,001 |
| Fraction 8 | 4903 | 21 ± 0,8 | 5 | n.s. | n.s. | 22 ± 0,1 | 16 | p<0,01 | p<0,001 |
| Fraction 9 | 4921 | 29 ± 0,6 | 45 | p<0,001 | p<0,001 | 29 ± 0,6 | 53 | p<0,001 | n.s. |
| Fraction 10 | 7500 | 21 ± 0,5 | 5 | n.s. | n.s. | 30 ± 0,6 | 58 | p<0,001 | n.s. |
| Fraction 11 | 7890 | 22 ± 0,5 | 10 | p<0,05 | p<0,05 | 29 ± 0,4 | 53 | p<0,001 | n.s. |
| SP 54 | 6600 | 20 ± 0,4 | 0 | n.s. | -- | 27 ± 0,5 | 42 | p<0,001 | -- |

(a) : % prolongation

Les résultats précédents montrent la nette potentialisation des propriétés antithrombotique et hypolipémiante de l'association de l'invention.

Ainsi, la fraction de l'exemple 1 administrée seule, par la voie intraduodénale, à la dose de 400 mg/kg, provoque un allongement de 60 % du temps de céphaline activée (TCA). En association avec 30 mg/kg d'acide citrique, l'allongement du TCA est porté à 240 %. L'étude toxicologique a mis en évidence la bonne tolérance de la composition thérapeutique suivant l'invention. En effet, sur ce plan, il n'a été constaté aucun phénomène de potentialisation. Les différentes études effectuées sur la toxicité aigue, chronique et retardée, ainsi que sur la tolérance locale et générale, n'ont mis en lumière aucune réaction anormale, aucune perturbation dans les contrôles biologiques, les courbes de croissance et les examens macroscopiques et microscopiques effectués après sacrifice.

Le médicament de l'invention peut être présenté sous les formes administrables par les voies orale, parentérale, rectale et locale. Chaque dose unitaire contient avantageusement de 0,030 g à 0,250 g de SP 54 ou une de ses fractions en association avec une quantité d'un acide organique allant de 0,050 g à 0,250 g.

Ainsi, les proportions de l'acide organique, dans le principe actif, peuvent varier, dans une large fourchette, de 20 % à 800 %, la proportion préférée étant comprise entre 80 % et 120 %. Les doses administrables journellement, exprimées en SP 54 ou ses fractions, peuvent varier de 0,030 g à 1,000 g en fonction de la gravité et de l'affection traitée.

On donnera, ci-après, à titre d'exemple non limitatif, quelques formulations pharmaceutiques du médicament de l'invention.

1 - Comprimés

. couche inférieure (Principe actif)

| | |
|---|---|
| Fraction de l'exemple 1................. | 250 mg |
| Lactose................................. | 50 mg |
| Cellulose excipient..................... | 60,1 mg |
| Magnésium stéarate...................... | 9 mg |
| Silicium dioxyde colloïdal.............. | 0,8 mg |
| Indigotine, laque aluminique............ | 0,1 mg |

Pour une couche terminée à 370 mg.

. couche intermédiaire (couche neutre)

| | |
|---|---|
| Lactose................................ | 132 mg |
| Cellulose excipient.................... | 109,7 mg |
| Magnésium stéarate..................... | 7,5 mg |
| Silicium dioxyde colloïdal............. | 0,6 mg |
| Jaune orangé S, laque aluminique...... | 0,2 mg |

Pour une couche terminée à 250 mg.

. couche supérieure (couche acide citrique)

| | |
|---|---|
| Acide citrique cristallisé............ | 250 mg |
| Cellulose excipient................... | 84,8 mg |
| Carboxyméthylcellulose................ | 5 mg |
| Magnésium stéarate.................... | 10 mg |
| Jaune de quinoléine, laque aluminique. | 0,2 mg |

Pour une couche terminée à 350 mg.

Pour un comprimé triple couche nu terminé à 970 mg

Pour chacune des formes précédemment citées, on peut réaliser un pelliculage gastrosoluble ou un pelliculage gastrorésistant dont les formules sont les suivantes :

suivantes :

- Pelliculage gastrosoluble

| | |
|---|---|
| Polyméthylacrylate de butyle et diméthylaminoéthyle, granulé............ | QS |
| Isopropanol pharmaceutique industriel... | QS |
| Acétone.............................. | QS |
| Phtalate de dibutyle.................. | QS |
| Acide silicique....................... | QS |

- Pelliculage gastrorésistant

| | |
|---|---|
| Polyméthylacrylate anionique, type 1, Poudre................................ | QS |
| Isopropanol pharmaceutique, pharmaceutique industriel......................... | QS |
| Acétone.............................. | QS |
| Phtalate de dibutyle.................. | QS |

2 - Gélules

| | |
|---|---|
| Fraction de l'exemple 4.................. | 0,100 g |
| Acide citrique cristallisé................ | 0,100 g |
| Excipient.................... Q. S. P..... | 1 gélule |

3 - Solutés injectables

| | |
|---|---|
| Fraction de l'exemple 7.................. | 0,100 g |
| Acide citrique cristallisé................ | 0,050 g |
| Solvant isotonique........... Q. S. P..... | 1 ampoule de 2 ml. |

4 - Pommade

| | |
|---|---|
| Fraction de l'exemple 11.................. | 0,150 g |
| Acide citrique cristallisé................ | 0,050 g |
| Excipient.................... Q. S. P..... | 1 tube de 30 g. |

Par leur action, sur les différents stades de la coagulation sanguine et de la fibrinolyse, ces médicaments peuvent être utilisés avec profit, en cures prolongées et répétées, dans le traitement préventif ou curatif d'accidents thromboemboliques veineux ou artériels dans les cas de phlébites, embolies pulmonaires et autres situations cliniques du même type, angines de poitrine, infarctus du myocarde, artérites chroniques des membres inférieurs, troubles circulatoires superficiels et ulcères des jambes.

Ils peuvent également être utilisés pour prévenir les thromboses dans les circuits extra-corporels (hémodialyses rénales).

En outre, leur action sur la lipoprotéine-lipase (facteur clarifiant) les rend particulièrement utiles dans le traitement des dyslipémies athérogènes.

Enfin, ces médicaments peuvent également être utilisés avec profit dans le traitement de certaines maladies inflammatoires, telles que polyarthrite rhumatoïde, arthroses et ostéoarthrites.

REVENDICATIONS

1. Composition thérapeutique ayant notamment une activité antithrombotique et lipolytique, caractérisée en ce qu'elle comprend, à titre de principe actif, une association de polysulfates de xylane et d'un acide organique.

2. Composition thérapeutique suivant la revendication 1, caractérisée en ce que le polysulfate de xylane est le SP 54.

3. Composition thérapeutique suivant la revendication 1 ou 2, caractérisée en ce que le polysulfate de xylane est une fraction du SP 54.

4. Composition suivant la revendication 1, caractérisée en ce que l'acide organique est de préférence, un acide di- ou polycarboxylique choisi parmi les acides citrique, isocitrique, tartrique, malique ou ascorbique.

5. Composition thérapeutique suivant l'une quelconque des revendications prédédents, caractérisée en ce qu'elle est présentée sous forme de doses unitaires contenant chacune de 0,030 g à 0,250 g de polysulfate de xylane et de 0,050 g à 0,250 g de l'acide organique, le principe actif étant associé à un véhicule pharmaceutiquement acceptable.

R E V E N D I C A T I O N S pour l'état contractant AT

1. Procédé de préparation d'une composition thérapeutique ayant notamment une activité antithrombotique et lipolytique, caractérisé en ce que l'on associe, à titre de principe actif, des polysulfates de xylane et un acide organique.

2. Procédé suivant la revendication 1, caractérisé en ce que le polysulfate de xylane est le SP 54.

3. Procédé suivant la revendication 1 ou 2, caractérisé en ce que le polysulfate de xylane est une fraction du SP 54.

4.Procédé suivant la revendication 1, caractérisé en ce que l'acide organique est un acide di- ou polycarboxylique choisi parmi les acides citrique, isocitrique, tartrique, malique ou ascorbique.

5. Procédé suivant l'une quelconque des revendications 1 à 4, caractérisé en ce que la composition est présentée sous forme de doses unitaires contenant chacune de 0,030 g à 0,250 g de polysulfate de xylane et de 0,050 g à 0,250 g de l'acide organique, le principe actif étant associé à un véhicule pharmaceutiquement acceptable.

Office européen des brevets

**RAPPORT DE RECHERCHE EUROPEENNE**

0189696

Numéro de la demande

EP 85 40 2522

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-3 658 969 (J.RENIE et al.)<br>--- | | A 61 K 31/725//<br>(A 61 K 31/725<br>A 61 K 31:19 ) |
| A | EP-A-0 125 152 (SANOFI)<br>--- | | |
| A | CHEMICAL ABSTRACTS, vol. 81, 1974, page 82, no. 10334y, Columbus, Ohio, US; St.K. BARTSOKAS et al.: "Synergy of a heparinoid and promethazine hydrochloride against liver cell necrosis", & RES. EXP. MED. 1974, 162(3), 235-41<br>--- | | |
| A | CHEMICAL ABSTRACTS, vol. 92, 1980, page 60, no. 209135a, Columbus, Ohio, US; G. Kindness et al.: "Synergistic inhibition by antithrombin III and heparin-like compounds of thrombin-indiced platelet aggregation", BIOCHEM. SOC. TRANS. 1980, 8(1), 85-6<br>--- | | DOMAINES TECHNIQUES RECHERCHES (Int. Cl.4)<br><br>A 61 K |
| A | US-A-4 167 562 (H.R. EVERS)<br>----- | | |

Le présent rapport de recherche a été établi pour toutes les revendications

| Lieu de la recherche | Date d'achèvement de la recherche | Examinateur |
|---|---|---|
| LA HAYE | 18-03-1986 | LENSEN H.W.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82